# EUROPEAN PATENT SPECIFICATION

(11) **EP 2 586 485 B1**
(45) Date of publication and mention of the grant of the patent: **14.03.2018**
(21) Application number: 12187237.8
(22) Date of filing: 04.10.2012
(51) Int. Cl.: A61M 25/10, F16H 25/20

(54) **Actuating mechanism for fluid displacement and pressurizing device**
Betätigungsmechanismus für Flüssigkeitsverdrängungs- und Druckbeaufschlagungsvorrichtung
Mécanisme d'actionnement pour déplacement de fluide et dispositif de pressurisation

(30) Priority: 31.10.2011 US 201113285662
(43) Date of publication of application: 01.05.2013
(73) Proprietor: Atrion Medical Products, Inc., Arab, AL 35016 (US); Kanner, Rowland W., Guntersville, AL 35976 (US); Davis, Richard M., Huntsville, AL 35803 (US); Roberts, Brian A., Owens Cross Roads, AL 35763 (US)
(72) Inventor: Kanner, Rowland W., Guntersville, AL 35976 (US); Davis, Richard M., Huntsville, AL 35803 (US); Roberts, Brian A., Owens Cross Roads, AL 35763 (US)
(74) Representative: Franks & Co Limited

(56) References cited:
- EP-A1- 2 186 540
- US-A- 5 713 242
- US-A1- 2002 133 116
- US-A1- 2012 067 204

## Description

### Background

This invention relates to fluid pressurizing devices for balloon catheters or the like, and more particularly relates to an improved device for actuating a screw plunger and monitoring the resulting fluid pressurization.

Fluid pressurization devices adapted for selectively applying and relieving a measured pressure on a closed volume of fluid have been developed for use in inflation and deflation of a balloon catheter used in angioplasty balloon procedures interiorly of blood vessels, or other types of balloon catheterization procedures. For example, U.S. Patent No. 4,838,864 discloses a syringe device which inflates and deflates a catheterized balloon. The device uses a manually-operated screw plunger to achieve or maintain specific balloon pressure, and the pressure is monitored using an associated pressure gauge. Improved syringing and pressurization control is also described in U.S. Patent Nos. 5,168,757; 5,713,242; and 6,796,959, all three of which are owned by the assignee of the present invention. These patents disclose quick release mechanisms which enable rapid advancement of a plunger and alternatively allow threaded engagement with the screw plunger to achieve precise control during final pressurization of a balloon catheter. Improved syringing and pressurization control is also described in European Patent No. EP2186540. This patent discloses a release mechanism where the control surface which is pressable to cause a nut member to disengage the plunger assumes the form of a lever on a nut member. A cross-sectional view of the device which is disclosed in U.S. Patent No. 6,796,959 is shown generally in Figure 1 herein (Figure 1 herein corresponds to Figure 4 of the '959 patent). As shown, the device 40 provides that a piston 48 is engaged with a plunger 68 inside a fluid displacement chamber 44 that is provided in a cylindrical syringe body or housing 42. A pressure gauge assembly 58 is threadably engaged with the housing 42, and the end 64 of the housing 42 is configured for engagement with a hose 54, such as a hose that is connected to a balloon catheter structure. The piston 48 has a sealing member 50 thereon which seals with an internal wall 72 of the housing 42. The plunger 68 can be retracted to pull fluid through the hose 54 into the device 40, and can be extended (or pushed in) to push fluid out of the device 40 and into the hose 54.

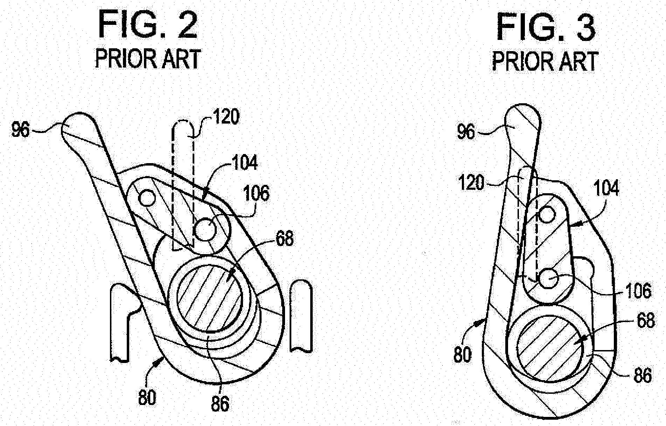

The plunger 68 can also be locked in place to prevent being pushed or pulled (i.e., to prevent macro movement of the plunger 68). Specifically, the device 40 includes a nut member 80 which can be moved into and out of engagement with a threaded portion 86 of the plunger 68. When the nut member 80 is not engaged with the threaded portion 86 of the plunger 68, as shown in Figure 2, the plunger 68 can be extended or retracted (i.e., pushed or pulled) using macro movements. In contrast, when the nut member 80 is engaged with the threaded portion 86 of the plunger 68, as shown in Figure 3, the plunger 68 is locked in place against macro movements, and can only be translated by using micro movements (i.e., by turning a knob 74 of the plunger 68).

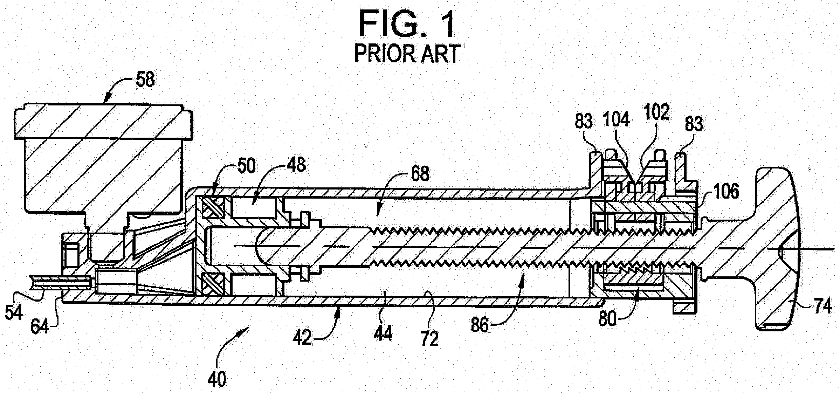

To facilitate movement of the nut member 80, there are two link members 102, 104 which are engaged with the nut member 80 and which are retained on the device 40 by a pivot pin 106. The link members 102, 104 are positioned and configured to operate in tandem. As shown in Figures 2 and 3, the nut member 80 also includes a lever or grip portion 96 which can manipulated in order to shift the nut member 80, causing the nut member 80 to move into and out of engagement with the threaded portion 86 of the plunger 68. In order to move the nut member 80 into or out of engagement with the threaded portion 86 of the plunger 68, the link members 102, 104 must clear past ribs 120 (see Figures 2 and 3) which are provided on flanges 83 (see Figure 1) which extend from the housing 42. Because the amount of force which is required to cause the link members 102, 104 to move past the ribs 120 depends on a plurality of part dimensions and tolerances, maintaining uniform detenting forces from one unit to the next, and from product line to product line, has proven challenging.

Another difficulty that can occur from time to time involves user convenience. Some users have found difficultly in reaching and engaging the lever 96 of the nut member 80 due to a combination of the manipulation required to perform the action and the operator's hand locations during certain procedures with the device 40, such as when the plunger 68 has been fully extended for vacuum and has to be locked into place. In such instances, the operator's hands can end up too far from the lever 96 to operate it without changing grip. During a disengage-withdraw plunger-reengage manipulation sequence (as occurs while drawing vacuum to pull down an interventional balloon after operation at pressure), users are forced to traverse the ribs 120 on the housing 42 twice by going one way and then back again the other direction in order to reengage the threaded plunger and hold it in a withdrawn position. Having to traverse the ribs 120 twice also occurs when manipulating to go from full vacuum or zero pressure to repressurization of the balloon.

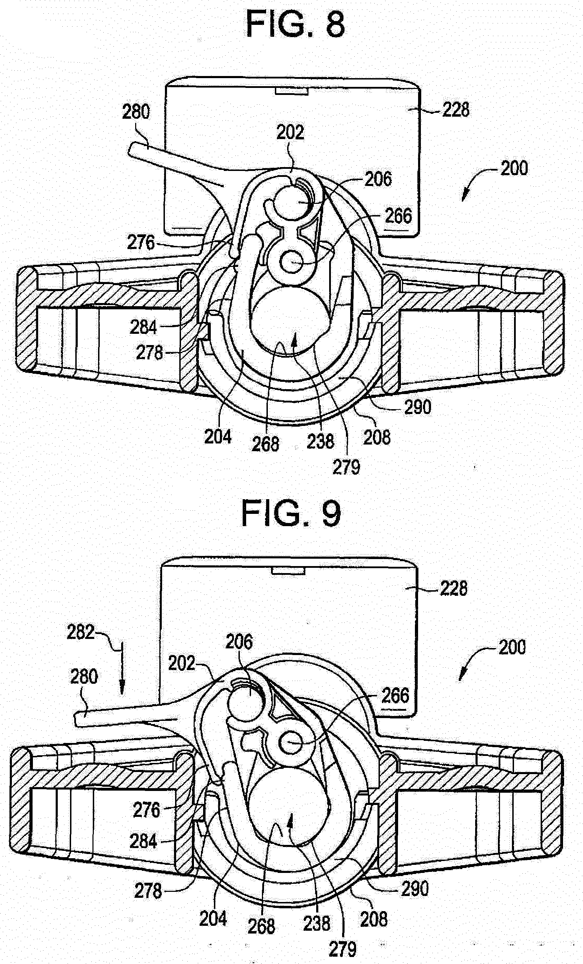

Another disadvantage of the device 40 shown in the '959 patent relates to assembly. As shown in Figures 8-11 of the '959 patent, assembly requires alignment and sliding of the first link member 102 into the nut member 80, followed by upside down insertion of the second link member 104 into the nut member 80, and then 180 degree rotation of the second link 104 to align it with the first one 102. This requires manipulation and assembly time. Additionally, due to the need for free and independent rotation of the two link members 102, 104, it is not possible to be certain that these link members 102, 104 always stay in alignment with one another or are properly located in the nut member 80 to receive the pivot pin 106 during assembly.

Furthermore, precise longitudinal alignment is not generally possible with regard to the two link members 102, 104 and the nut member 80, and to both the pivot pin 106 and the threads 86 on the plunger 68. Specifically, each link member 102, 104 is free to pivot out of alignment slightly with the other and consequently allow the nut member 80 to slightly twist or deflect out of alignment with the plunger's axis during engagement and disengagement manipulations. Such twist is possible even though plunger 68 and pivot pin 106 are maintained in strict parallel alignment to one another. This undesirable deflection offers potential for a deleterious effect upon mating thread components resulting in uneven loading and occasional chipping of the most highly loaded threads during disengagement during maximum pressure use conditions. With relation to the device shown in EP2186540, a simplified design is achieved and therefore the tooling of the nut member for easier manufacture of the nut member while having minimal impact upon the design and ease of manufacture of the unitary link.

### Objects and Summary

An object of an embodiment of the present invention is to provide an improved fluid displacement device.

Another object of an embodiment of the present invention is to provide an improved process for assembling a fluid displacement device.
Still another object of an embodiment of the present invention is to provide a fluid displacement device which is relatively easy to assemble.

Briefly, and in accordance with at least one of the foregoing objects, the present invention provides a toggle locking mechanism for a fluid displacement device as claimed in claim 1. The present invention also provides a fluid displacement device as claimed in claim 10, particularly for use of the device to pressurize and depressurize catheters, or inject fluid, or aspirate fluid, or the like. The device includes a plunger which is displaceable through a housing, and an actuating mechanism in the form of a toggle locking mechanism which engages the plunger. The actuating mechanism includes a nut member and a unitary link member that provides an integral spring. The integral spring of the unitary link member provides that the nut member is biased into engagement with the threaded portion of the plunger. As such, the plunger is normally in the "locked" position, thereby preventing macro movements (i.e., pushing or pulling) of the plunger but allowing micro movements (i.e., turning) of the plunger. The actuating mechanism is configured to provide a press-to-release feature wherein the plunger is instantly releaseable by simply depressing a control surface, such as a control surface on the nut member or unitary link member, to overcome a restorative spring force provided by the integral spring of the unitary link member. Subsequently, the plunger can be translated using macro movements (i.e., by pushing or pulling the plunger).

Another aspect of the present invention provides a method of assembling a fluid displacement device as set out in claim 11. The assembly method includes assembling an actuating mechanism of the device by engaging a unitary link member with a nut member, whereby integral spring force of the unitary link member tends to hold the unitary link member in place on the nut member. A pin is then engaged with the unitary link member and the nut member, thereby forming an assembly. This assembly is then inserted in an opening in the housing and another pin is used to retain the assembly on the device.

The structure and method of assembling the fluid displacement device provide several advantages, as will be described more fully later hereinbelow.

### Brief Description of the Drawings

The organization and manner of the structure and operation of the invention, together with further objects and advantages thereof, may best be understood by reference to the following description, taken in connection with the accompanying drawings, wherein like reference numerals identify like elements in which:
Figure 1 is a cross-sectional view of a prior art fluid displacement device, specifically that which is disclosed in U.S. Patent No. 6,796,959;
Figures 2 and 3 are enlarged views which illustrate two different positions of an actuating mechanism of the fluid displacement device which is shown in Figure 1;
Figure 4 is a perspective view of a fluid displacement device which is in accordance with an exemplary device;
Figure 5 is a cross-sectional view of the fluid displacement device shown in Figure 4;
Figure 6 is an exploded perspective view of the fluid displacement device shown in Figures 4 and 5;
Figure 7 is an enlarged exploded perspective view of a portion of the fluid displacement device shown in Figures 4-6, specifically an actuating mechanism thereof;
Figure 8 is a partial cross-sectional view of the fluid displacement device shown in Figures 4-6, showing the actuating mechanism biased into engagement with a plunger of the device;
Figure 9 is a partial cross-sectional view of the fluid displacement device shown in Figures 4-6, showing the actuating mechanism being actuated such that it has moved out of engagement with the plunger;
Figure 10 is a front, elevational view of a carrier member component of the fluid displacement device shown in Figures 4-6;
Figure 11 is a rear, elevational view of the carrier member;
Figure 12 is a top, plan view of the carrier member;
Figure 13 is a side, elevational view of the right side of the carrier member;
Figure 14 is a side, elevational view of the left side of the carrier member;
Figure 15 is a cross-sectional view of the carrier member, taken along line 15-15 of Figure 11;
Figure 16 is a cross-sectional view of the carrier member, taken along line 16-16 of Figure 11;
Figure 17 is a cross-sectional view of the carrier member, taken along line 17-17 of Figure 14;
Figure 18 is a partial cross-sectional view of the actuating mechanism of the fluid displacement device shown in Figures 4-6, showing an internal rib feature;
Figure 19 is a perspective view of a fluid displacement device which is in accordance with an embodiment of the present invention;
Figure 20 is an exploded perspective view of the fluid displacement device shown in Figure 19;
Figure 21 is an enlarged exploded perspective view of a portion of the fluid displacement device shown in Figures 19-20, specifically an actuating mechanism thereof;
Figure 22 is a partial cross-sectional view of the fluid displacement device shown in Figures 19-20, showing the actuating mechanism biased into engagement with a plunger of the device;
Figure 23 is a partial cross-sectional view of the fluid displacement device shown in Figures 19-20, showing the actuating mechanism being actuated such that it has moved out of engagement with the plunger; and
Figure 24 is an enlarged exploded perspective view of alternative actuating mechanism.

### Description

While the invention may be susceptible to embodiment in different forms, there are shown in the drawings, and herein will be described in detail, specific embodiments with the understanding that the present disclosure is to be considered an exemplification of the principles of the invention, and is not intended to limit the invention to that as illustrated and described herein.

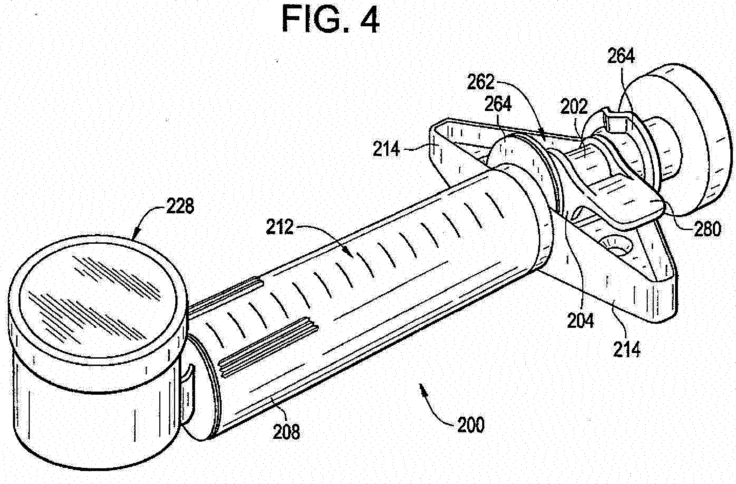

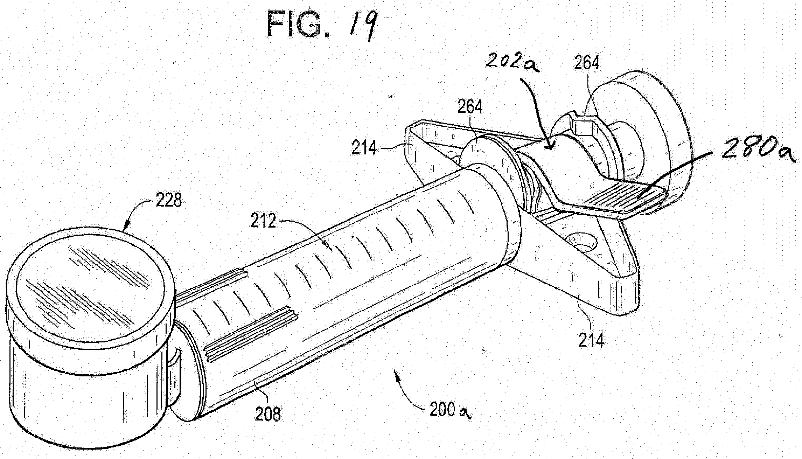

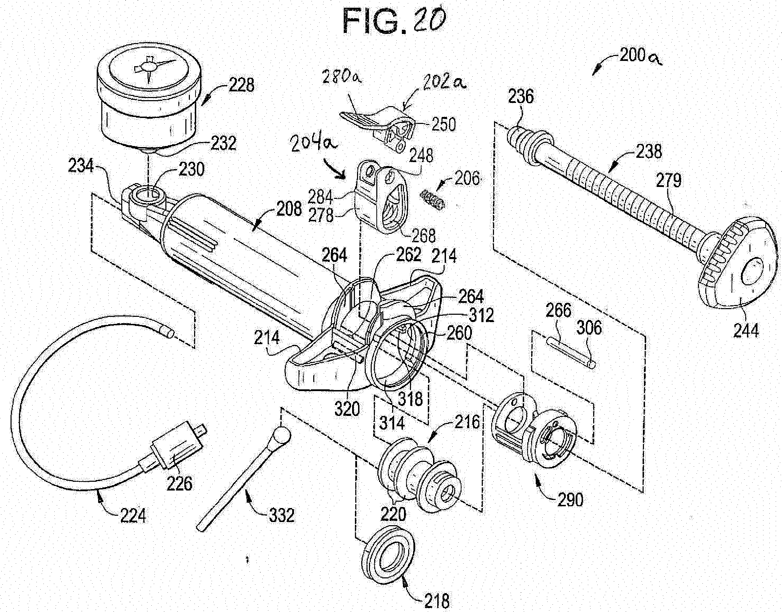

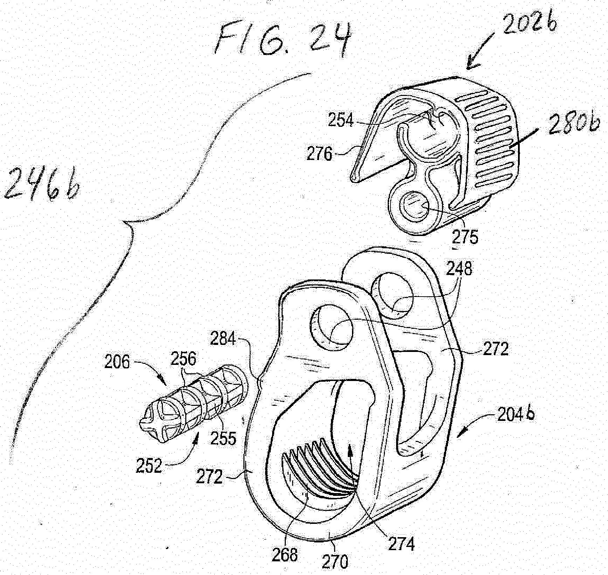

One specific example (not claimed) is shown in Figures 4-6 while a specific embodiment of the present invention as claimed is shown in Figures 19 and 20. Still another variation of these two is shown in Figure 24. The example shown in Figures 4-6, which does not form part of the invention as claimed, will be described first, and then differences between that example and the other variations, which form part of the invention as claimed, will be discussed.

A first, specific example comprises a fluid displacement device 200 (see Figures 4-6) which utilizes all the same components of the device disclosed in U.S. Patent No. 6,796,959, except for replacing the nut member 80 and link members 102, 104 of the '959 device with a unitary link 202, a nut member 204, and a pivot pin 206.

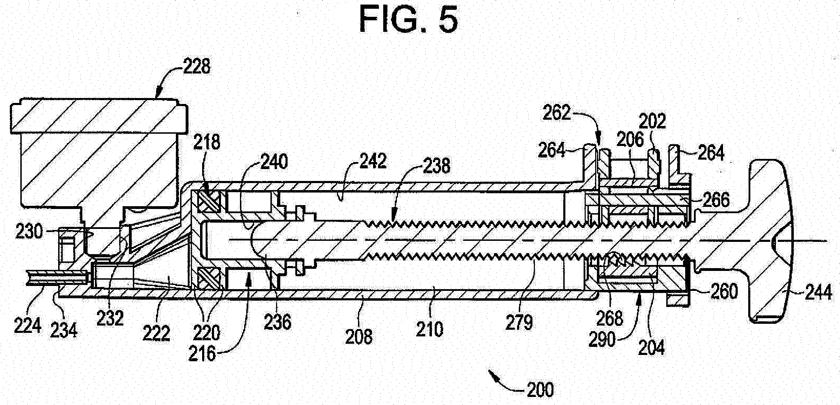

As shown in Figures 4-6, the fluid displacement device 200 which is in accordance with a specific example has a generally cylindrical syringe body or housing 208 which provides a fluid displacement chamber 210 (see specifically Figure 5). Preferably, the housing 208 is transparent thereby facilitating the viewing of fluid in the fluid displacement chamber 210 during fluid aspiration or dispensing. The housing 208 is formed of plastic, and may be molded from polycarbonate or another type of resin. The housing 208 has volumetric indicia thereon, such as at location 212 indicated in Figure 4, so that a physician can readily determine the volume of fluid contained in the chamber 210. As shown, handles 214 are provided on the housing 208 to facilitate gripping of the device 200 and operation thereof.

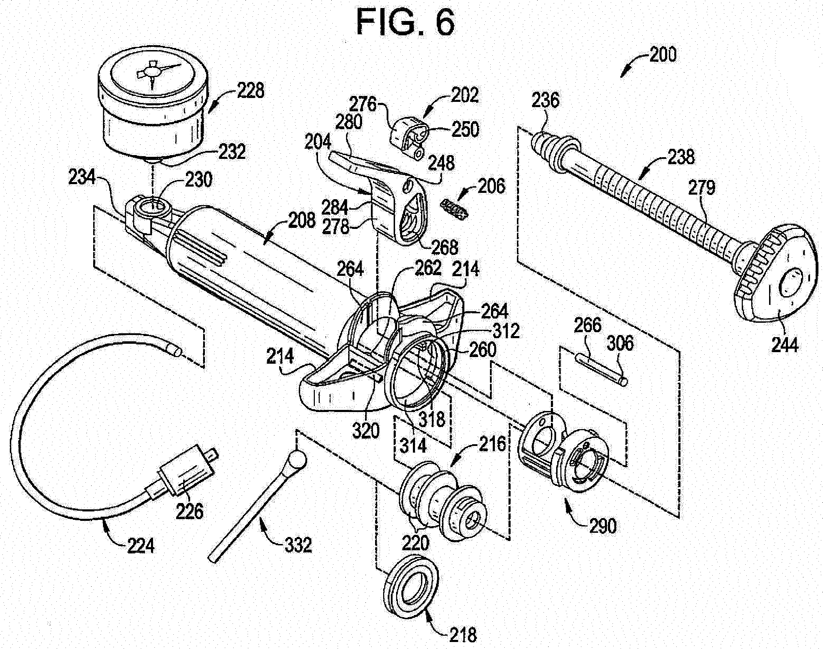

A piston 216 is slidably displaceable within the fluid displacement chamber 210 (i.e. in the housing 208). A sealing member 218, such as a quad ring, is disposed on the piston 216. As shown in Figure 5, preferably the sealing member 218 is disposed between two walls 220 on the piston 216. As will be described more fully later hereinbelow, a lubricant may be used to place the sealing member 218 on the piston 216. The sealing member 218 is configured for pressure retention and the prevention of leakage of fluid past the piston 216 in the fluid displacement chamber 210. The housing 208 includes a fluid conduit 222 which is in communication with the fluid displacement chamber 210. The fluid conduit 222 is also in communication with a hose 224 that is connected to, for example, balloon catheter structure (not shown), a fluid supply reservoir (not shown) or some other suitable structure depending on the application. As shown in Figure 6, a connector 226 may be provided on the hose 224, between the fluid conduit 222 and the balloon catheter.

As shown in Figure 5, the fluid conduit 222 also communicates with a pressure gauge assembly 228. Preferably, the pressure gauge assembly 228 is engaged directly with the housing 208, such as threadably engaged with a threaded bore 230 (see Figures 5 and 6) thereon. To this end, the pressure gauge assembly 228 is provided with a threaded portion 232 which threadably engages the threaded bore 230 on the housing 208. An adhesive may be provided between the threaded portion 232 and the threaded bore 230 to lockingly and sealingly engage the parts together. As shown in Figures 5 and 6, the threaded bore 230 is provided generally proximate an end 234 of the housing 208 such that, when installed, the pressure gauge assembly 228 is disposed generally proximate a distal end of the device. The pressure gauge assembly 228 is configured to provide a physician with an indication of the pressure in the fluid conduit 222 and balloon catheter. The pressure gauge assembly 228 can be of any suitable type for either angioplasty or for other employment of the device 200. The fact that the device 200 is configured such that the pressure gauge assembly 228 engages directly with the housing 208 provides increased visibility proximate the distal end of the device. Such visibility becomes important when a physician is to purge all the air bubbles from the fluid displacement chamber 210 before using the device 200 to dispense fluid. Alternatively, the device 200 can be configured such that the pressure gauge assembly 228 engages a clamping cover such as is shown and described in U.S. Patent No. 5,713,242. As best shown in Figure 5, the piston 216 is mounted on an unthreaded pilot nose end 236 of a plunger 238. The pilot nose end 236 is configured to freely rotate in a central journal cavity 240 of the piston 216. The piston 216 is mounted on the pilot nose end 236 of a plunger 238 in a snap-action, interference coupling which prevents the pilot nose end 236 from withdrawing or backing out of the central journal cavity 240 of the piston 216 when the plunger 238 is retracted to aspirate fluid into the fluid displacement chamber 210. The pilot nose end 236 is freely rotatable relative to the coupled piston 216. As such, when the plunger 238 is rotated (i.e., during micro movements), the piston 216 does not rotate along with the plunger 238, but generally advances or retracts linearly in the housing 208. As shown in Figures 4-6, the plunger 238 preferably includes an integral palm knob 244 which facilitates movement of the plunger 238 as will be described in more detail hereinbelow.

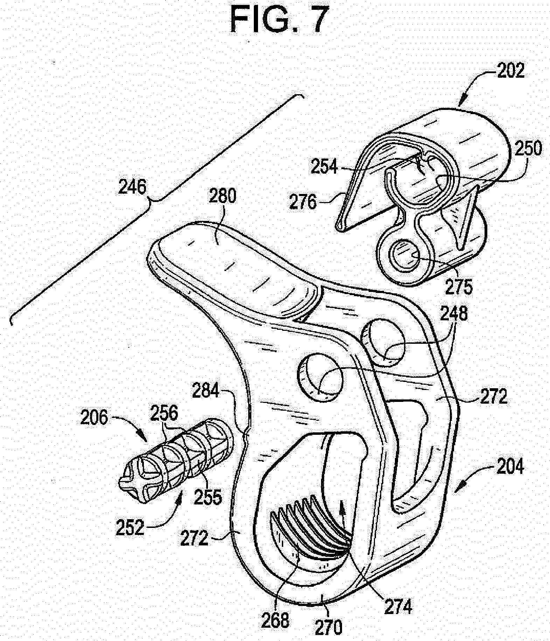

The device includes an actuating mechanism 246 which is normally biased into engagement with the plunger 238, but which can be actuated out of engagement with the plunger 238. When the actuating mechanism 246 (see Figure 7) is biased into engagement with the plunger 238, macro movements of the plunger 238 are not generally possible, only micro movements are. When macro movements are prevented, the plunger 238 cannot be pushed into or pulled out of the housing 208 (i.e., to displace a large amount of fluid quickly). Instead, the plunger 238 can only be translated by rotating its knob 244 (i.e., to displace fluid slowly, with more precision). On the other hand, when the actuating mechanism 246 is actuated out of engagement with the plunger 238, macro movements of the plunger 238 are possible. When macro movements are possible, the plunger 238 can be pushed into or pulled out of the housing 208.

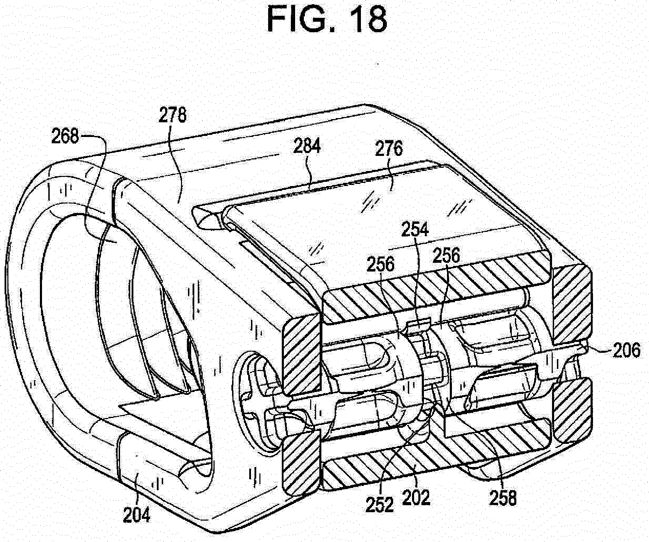

As shown in, for example, Figure 7, the actuating mechanism 246 comprises a unitary link 202, a nut member 204, and a pivot pin 206. The pivot pin 206 extends through holes 248 on the nut member 204 and though a passageway 250 in the unitary link 202, thereby effectively retaining the unitary link 202 on the mating nut member 204 and forming an assembly. As shown in Figures 7 and 18, the pivot pin 206 is preferably provided with a reduced diameter middle portion 252 which receives a central rib 254 within the unitary link 202 which locks the pivot pin 206 in place relative to the unitary link 202. Specifically, the pin 206 has a cruciform shape, with lower cruciform members 255 being provided between two central disk features 256. These lower cruciform members 255 effectively form a groove 258 on the pivot pin 206, wherein the raised rib 254 in the unitary link 202 is configured to catch in the groove 258 on the pivot pin 206 and hold the pivot pin 206 in place.

As shown in Figure 4 and 5, the overall assembly is disposed in the housing 208, generally near a rearward end 260 of the housing 208. Specifically, as shown in Figures 4-6, the actuating mechanism 246 is preferably disposed in an aperture 262 in the housing 208, where the aperture 262 is provided between a pair of spaced flanges 264, and the actuating mechanism 246 is mounted on the device 200 viz-a-viz a pin 266, which is preferably made of metal.

As viewed in Figures 5-7, the nut member 204 has threads 268 which are formed in a lower base portion 270, and the nut member 204 includes an upstanding, bifurcated portion which provides a pair of spaced, elongate arm or mounting portions 272 (see specifically Figure 7). The mounting portions 272 in effect define carriage structure, and are generally integral with the threads 268. Each of the mounting portions 272 has an aperture 274 therein, through which the plunger 238 extends (see specifically Figures 5, 8 and 9), as well as a hole 248 for receiving the pivot pin 206.

Figure 8 illustrates how it is that the nut member 204 is biased into engagement with the plunger 238. Specifically, a spring finger portion 276 of the unitary link 202 engages an external surface 278 of the nut member 204, and this contact along with the rigidity of the spring finger portion 276 of the unitary link 202 generates a restorative spring force which tends to keep the nut member 204 in the position shown in Figure 8, wherein the threads 268 on the nut member 204 are engaged with threads 279 of the plunger 238. In this position, the plunger 238 cannot be pushed into or pulled out of the housing 208 using macro movements. Instead, the plunger 238 must be translated using only micro movements, i.e. by turning its knob 244, causing the threads 279 of the plunger 238 to worm along the threads 268 of the nut member 204. As described above, the pilot nose end 236 of the plunger 238 is configured to freely rotate in the central journal cavity 240 of the piston 216. Hence, rotation of the plunger 238 while the threads 268 of the nut member 204 are engaged with the threaded portion 279 of the plunger 238 generally does not cause the piston 216 to rotate in the fluid displacement chamber 210 in the housing 208, but instead allows the piston 216 to merely slide in the housing 208.

To provide the integral spring feature (i.e., the spring finger 276) of the unitary link 202, the unitary link 202 is made of a material that has both excellent compressive strength and good stiffness coupled with a high degree of elasticity to allow deflection without plastic deformation or breakage. Acetal resin, commercially known as Delrin or Celcon for instance, offers these properties, but to a lesser degree than do Nylon, Polypropylene or similar elastic materials. Acetel is often preferred for polymer spring applications due to its ability to withstand large degrees of deflection without plastic deformation or fracture along with its inherent lubricity, hardness and resistance to environmental stress, cracking and wear. In the application disclosed herein, the part must be able to tolerate pivoting and sliding against mating components of Nylon and metal for which Acetal is known to perform particularly well.

By virtue of the return spring force being sufficient to keep the device 200 in a plunger-engaged, default position, ribs (identified with reference numeral 120 in Figures 2 and 3, herein) such as is shown in U.S. Patents 5,713,242 and 6,796,959, and the additional user effort which is required to overcome them, are no longer necessary. When the nut member 204 is biased into engagement with the plunger 238 as shown in Figure 8, the nut member 204 is in an "overcenter" locking position which ensures that any slight motion of the nut member 204 which could be induced by fluid pressure in the fluid displacement chamber 210 imposed on piston 216 results in tighter engagement of the plunger 238 and nut member 204 rather than any tendency for loosening or disengagement thereof.

The nut member 204, and specifically the threads 268 thereof, is selectively disengageable from the plunger 238 in order to permit manual rapid displacement of the plunger 238 and piston 216, for example, for aspiration of saline solution into the fluid displacement chamber 210 from a fluid supply reservoir (not shown) which may be connected to the hose 224 or in which connector 226 may be immersed. The disengagement of the nut member 204 from the plunger 238 also enables rapid advancement of the plunger 238 and piston 216, for example, to discharge solution through the hose 224 to inflate an angiolplasty balloon (not shown) which has been previously positioned within a blood vessel or heart valve using a balloon catheter. Rapid retraction of the plunger 238 and piston 216 may also enable swift aspiration of fluid into the fluid displacement chamber 210 for rapid deflation of an angiolplasty balloon. Also, prior to connection to the catheter, the plunger 238 may be operated to insure that all air bubbles have been eliminated from the fluid which is contained in the fluid displacement chamber 210.

In order to facilitate the selective disengagement of the threads 268 of the nut member 204 with the threaded portion 279 of the plunger 238, as shown in Figure 9, the nut member 204 is provided as having a control surface such as a lever 280 (see Figures 4, 8 and 9), wherein disengagement of the nut member 204 is achieved by pressing down on the lever 280 (as represented by arrow 282 in Figure 9). Pressing down on the lever 280, as shown in Figure 9, causes the assembly to traverse, and causes the spring finger portion 276 of the unitary link 202 to ride along the external surface 278 of the nut member 204 and be deflected outwardly. Displacement of the nut member 204 is preferably guided along a translating motion between the positions shown in Figures 8 and 9. The motion of the nut member 204 in moving between the positions of Figures 8 and 9 is in actuality that of a diminishing curved path and, as such, is not truly linear. When the lever 280 of the nut member 204 is pressed down, the spring finger portion 276 of the unitary link 202 becomes flexed, generally creating a bias of the assembly back into the position shown in Figure 8. As such, releasing the lever 280 causes the assembly to return to the position shown in Figure 8. Figure 8 illustrates the normal, non-actuated position of the nut member 204 in which the threads 268 of the nut member 204 are biased into engagement with the threaded portion 279 of the plunger 238, thereby locking the plunger 238 with regard to macro movements. However, micro movements of the plunger 238 are possible by rotating its knob 244, causing the threads of the plunger 238 to worm along the threads 268 on the nut member 204.

In contrast, Figure 9 illustrates what occurs when a user presses down on the lever 280 of the nut member 204. As shown, this causes the nut member 204 to traverse, causing the threads 268 of the nut member 204 to become disengaged from the threaded portion 279 of the plunger 238. In such a position, the plunger 238 may be axially translated in the fluid displacement chamber 210 by pushing the plunger 238 forward into the housing 208, or by pulling the plunger 238 back out of the housing 208. In other words, pushing or pulling the plunger 238 is prevented when the nut member 204 is engaged with the plunger 238. However, pushing the lever 280 of the nut member 204 down causes the nut member 204 to disengage the plunger 238, thereby allowing the plunger 238 to be pushed or pulled.

Although only optional, the nut member 204 preferably includes a cam feature 284 as shown in Figures 6-9. When the lever 280 of the nut member 204 is pushed down, the spring finger 276 of the unitary link 202 must ride up on the cam 284, as shown in Figure 9. The cam 284 works to increase the closing force (i.e., the tendency for the overall actuating mechanism 246 to return to the position shown in Figure 8) and provide a definitive location to assure that the at-rest position (see Figure 8, wherein the lever 280 of the nut member 204 is not being pressed) is fully achieved by the nut member 204 rather than allowing this position to be indeterminate as may be the case without the cam feature. The cam feature 284 provides both auditory and tactile feedback to the user with regard to whether the nut member 204 is engaged with the plunger 238. To provide more definitive feedback, the cam feature 284 may be configured to provide a sharp step for spring finger 276 to ride over and subsequently return over. This provides that the spring finger 276 will snap over cam 284, providing more definitive auditory and tactile feedback to the user of the released or engaged position of threads 268 with plunger 238.

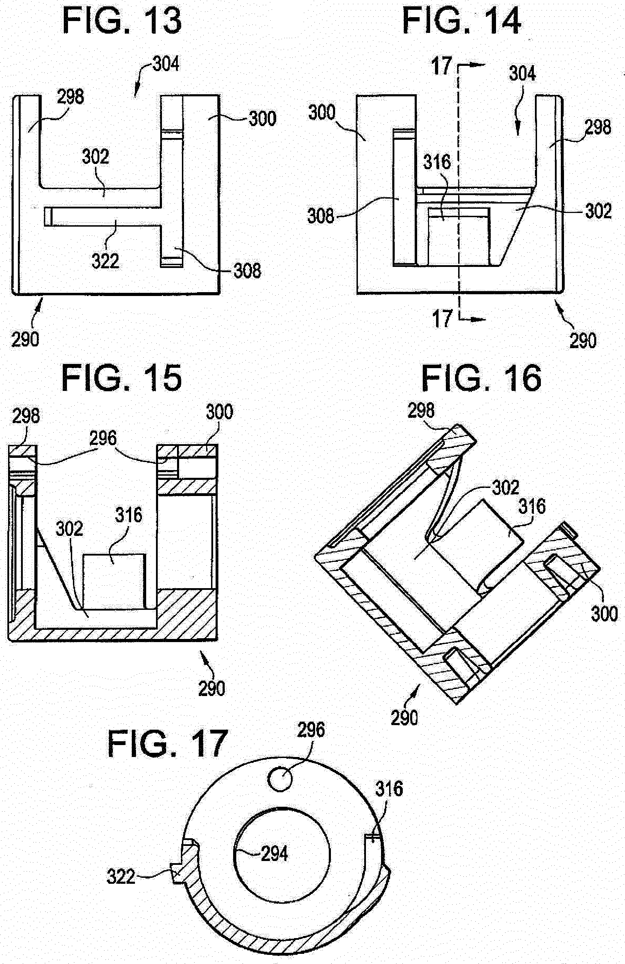

As shown in Figures 5, 6, 8 and 9, preferably the device 200 includes a carrier member 290 that is received in the housing 208, much like the device shown in U.S. Patent 6,796,959. As shown in Figures 6 and 10-17, the carrier member 290 may be provided in the form of a generally hollow, generally cylindrical part. The carrier member 290, like the housing 208, may be formed of plastic. In fact, the carrier member 290 and the housing 208 may be formed of the same resin.

As shown in Figures 5, 6, 10, 11, 15 and 17, the carrier member 290 has spaced-apart walls 298, 300, and the walls 298, 300 are provided with apertures 294 through which the ) plunger 238 extends, as well as apertures 296 which retain the pin 266 that secures the actuating mechanism 246 to the device 200. More specifically, the carrier member 290 supports the pin 266, and the pin 266 extends through a bore 275 in the unitary link 202, thereby providing that the pin 266 generally retains the actuating mechanism 246 on the device 200.

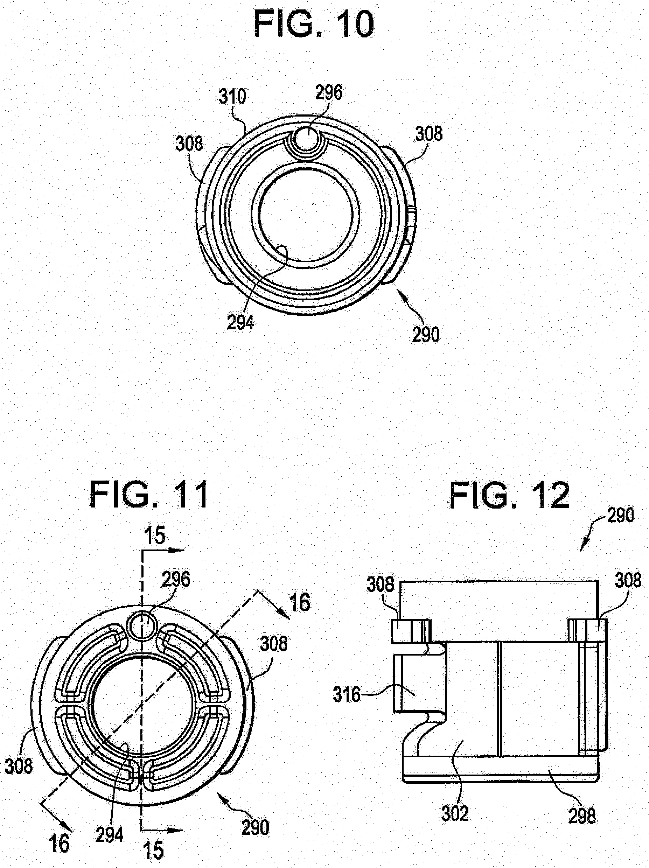

As shown in Figure 6, wall 298 defines the front surface of the carrier member 290 (see also Figure 10, which provides a view of the front of the carrier member 290), and wall 300 defines the rear surface of the carrier member 290 (see also Figure 11, which provides a view of the rear of the carrier member 290). A partial wall 302 extends between the front and rear walls 298 and 300 of the carrier member 290. A cut out 304 is defined between the walls 298 and 300, and the nut member 204 is disposed in the cut out 304 (see Figure 5). The pin 266 extends through the apertures 296 provided in the carrier member 290, and the carrier member 290 generally retains the pin 266 such that the pin 266 does not readily move axially. As shown in Figure 6, the pin 266 may be provided with a knurled end portion 306 which assists in preventing the pin 266 from translating axially once it is installed in the carrier member 290.

As shown in Figures 6 and 10-15, protruding surfaces 308 are provided on an external surface 310 of the carrier member 290, generally proximate the rear end of the carrier member 290. Corresponding inward facing flanges 312 are provided in the housing 208, at the rearward end 206 thereof, for receiving interruptions between protruding surfaces 308 provided on the carrier member 290. Preferably, the flanges 312 in the housing 208 and the interruptions between protruding surfaces 308 on the carrier member 290 provide that the carrier member 290 can be inserted in a bore 314 in the end 260 of the housing 208, and then rotated to secure the carrier member 290 in the housing 208. Specifically, the carrier member 290 and housing 208 are configured such that the carrier member 290 is installable through the rear portion of the housing 208 in the bore 314 in the housing 208 via a quarter-turn bayonet arrangement, wherein the carrier member 290 is axially inserted in the bore 314 and then given a one-quarter turn to lock the carrier member 290 in place.

The carrier member 290 includes a latching finger 316 which is formed as part of the partial wall 302 which extends between the front and rear walls 298, 300 of the carrier member 290. The latching finger 316 preferably engages an inwardly extending rib 318 provided on an internal wall of the housing 208 (see Figure 6). Specifically, the latching finger 316 engages and clears the inwardly extending rib 318 on the housing 208 in a manner which generally locks the carrier member 290 in place in the bore 314 in the housing 208. Preferably, the latching finger 316 and inwardly extending rib 318 are configured such that an audible noise is produced as the latching finger 316 engages and clears the inwardly extending rib 318. The noise is loud enough to provide an audible indication to an assembler of the device 200 that the carrier member 290 is fully installed in the bore 314.

Preferably, another inwardly extending rib 320 is provided on the internal wall of the housing 208 exactly opposite rib 318 (i.e. 180 degrees from rib 318, along the interior wall of the housing 208). A protruding surface 322 is provided on the external surface 310 of the carrier member 290, generally extending from protrusion 308 of the carrier member 290 toward the front end of the carrier member 290. When the carrier member 290 is inserted in the bore 314, the protruding surfaces 308 abut the proximal ends of ribs 318 and 320 (see Figures 5 and 8-10) of the housing 208 to prevent the carrier member 290 from installing too far axially into the bore 314. In other words, engagement of the protruding surfaces 308 with ribs 318 and 320 limits axial travel of the carrier member 290 in the bore 314 during installation. Once the protruding surface 308 contacts ribs 318 and 320, the carrier member 290 is rotated such that it becomes fully installed. Preferably, not only does the latching finger 316 of the carrier member 290 engage and clear the inwardly extending flange 318 on the housing 208, but the protruding surface 308 on the carrier member 290 engages behind the opposite inwardly extending flanges 312 on the internal surface of the housing 208. When the carrier member 290 is fully installed in the bore 314, the carrier member 290 cannot be readily axially withdrawn from the bore 314, nor can the carrier member 290 be readily rotated in either rotational direction relative to the housing 208. When the carrier member 290 is properly installed in the housing 208, the cut out 304 in the carrier member 290 is generally aligned with the aperture 262 in the housing 208.

The nut member 204, unitary link 202 and pin 206 (i.e., the actuating mechanism 246) is received in the cut out 304 in the carrier member 290 and the aperture 262 in the housing 208 such that the lever 280 of the nut member 204 generally extends out the aperture 262 in the housing 208 for easy access, as shown in Figures 4, 8 and 9. The carrier member 290 is securably engaged relative to the housing 208, and the pin 266 which secures the actuating mechanism 246 is retained by the carrier member 290.

The carrier member 290 is configured to withstand in-use forces as the plunger 238 is translated forward in the fluid displacement chamber 210. The carrier member 290 permits the device 200 to be assembled from the rear of housing 208 (i.e. is rear loaded), as will be described more fully later hereinbelow in connection with describing assembly of the device. Additionally, as shown in Figure 5, the carrier member 290 provides that a distance from a center line of the plunger 238 to an internal wall 242 of the housing 208 can be greater than a distance from the center line of the plunger 238 to the pin 266.

Still further, the design of the device 200, by including the carrier member 290, provides that many of the parts are universal such that they are compatible with devices of different sizes. Specifically, the nut member 204 and plunger 238 may be used in association with different sized devices, specifically devices with different sized fluid displacement chambers. The carrier member 290 shown in the Figures is designed to be used with fluid displacement chambers which are as large as that shown in the Figures or which are smaller than that shown in the Figures. Of course, a larger or smaller sized device can be provided than is depicted in the Figures, which are not to scale.

Despite all the advantages that are provided by having the device include the carrier member 290, the device can instead be provided as not having a carrier member, such as is disclosed in U.S. Patent No. 115,713,242. Regardless, the device 200 is configured such that a user need not change grip of the device 200 during use. The lever 280 of the nut member 204 just needs to be pressed to effect plunger 238 disengagement, and ribs (identified with reference numeral 120 in Figures 2 and 3) need not be overcome to move back and forth between a plunger-engaged and a plunger-disengaged position. Additionally, release of the lever 280 provides instant plunger 238 reengagement. By virtue of the return spring force provided by the spring finger 276 of the unitary link 202 being sufficient to keep the device 200 in a plunger-engaged default state (as shown in Figure 8), detents or ribs on the housing 208 and additional user effort required to overcome them are no longer necessary.

A method of assembling the device 200 will now be described, in the situation where the device does, in fact, include a carrier member 290 such as disclosed in U.S. Patent No. 6,976,959. To assemble the device 200, hose 224 may be affixed to the housing 208. Then, the pressure gauge assembly 228 is threadably engaged with the housing 208. Subsequently, the sealing member 218 is fit onto the piston 216. To facilitate this, a lubricant can be used as illustrated by applicator 332 shown in Figure 6. Then, the piston 216 is slipped into bore 314 in the housing 208 (i.e. from right-to-left in Figure 5). In other words, the piston 216 is rear loaded into the device 200. Then, the carrier member 290 is installed on the housing 208, also from the rear portion of the housing 208. As described above, this installation may be via a quarter-turn bayonet style installation wherein the carrier member 290 is axially inserted into the bore 314 and then is rotated a quarter-turn to lock the carrier member 290 in place relative to the housing 208. As described above, preferably the locking finger 316 on the carrier member 290 emits a snapping or clicking sound once the carrier member 290 is fully and properly installed. When the carrier member 290 is properly installed in the housing 208, the cut out 304 in the carrier member 290 is generally aligned with the aperture 262 in the housing 208. Subsequently, the actuating mechanism 246 can be installed in the aperture 262. However, before the actuating mechanism 246 is installed, the actuating mechanism 246 must be assembled.

To assemble the actuating mechanism 246, the unitary link 202 is clipped onto the nut member 204, and the pivot pin 206 is used to secure the unitary link 202 to the nut member 204. Even before the pivot pin 206 is used to secure the unitary link 202 to the nut member 204, the unitary link 202 tends to stay in place relative to the nut member 204 due to the spring force provided by the integral spring feature 276 of the unitary link 202. To secure the unitary link 202 to the nut member 204, the pivot pin 206 is pushed through one hole 248 on the nut member 204, though a passageway 250 in the unitary link 202, and into the other hole 248 in the nut member 204, thereby effectively forming an assembly. As shown in Figure 18, when the pivot pin 206 is in place, a central rib 254 in the unitary link 202 engages a reduced diameter middle portion 252 of the pivot pin 206, thereby locking the pivot pin 206 in place relative to the unitary link 202. Once the actuating mechanism 246 is assembled, the actuating mechanism 246 can be inserted into the aperture 262, and the pin 266 used to secure the actuating mechanism 246.

After the actuating mechanism 246 has been installed on the device 200, the lever 280 of the actuating mechanism 246 is pushed down, and the plunger 238 is axially inserted into the rear end 260 of the housing 208 into engagement with the piston 216. Specifically, the nose end 236 of the plunger 238 is inserted through bore 314, through the bore 274 provided in the nut member 204, and into snap-fit engagement with the piston 216 within the fluid displacement chamber 210. Thereafter, the lever 280 of the actuating mechanism 246 can be released, and device 200 is thereafter operable.

As described hereinabove, assuming the device is provided in accordance with U.S. Patent No. 6,976,959, and includes a rear-loading carriage member 290, the device is configured to be assembled from the rear. Specifically, both the piston 216 and carrier member 290 are installed in the rear of the housing 208. By providing that the device is assembled from the rear, the housing 208 can be provided as a single, integral piece. The front portion of the housing 208 can be molded as part of the housing 208, and no extra fittings or clamping covers need to be used to mount the gauge 228 to the housing 208. The gauge 228 can be engaged directly with the housing 208. Hence, the design has fewer parts and requires less labor than if the device were provided as being consistent with that which is shown in U.S. Patent No. 5,713,242. Additionally, there is no issue with regard to sealing the front of the device 200 because the front portion of the device 200 is integral with the remainder of the housing 208. Moreover, visibility at the front of the device 200 is improved, and this is advantageous to a user who is attempting to prime the device 200 before use by purging all the air bubbles from the chamber 210. By providing that the housing 208 is molded as a single piece, the housing 208 can be manufactured in a single molding operation, thereby reducing the cost of the components and the molds, creating less waste and streamlining the production and assembly process.

Furthermore, the design of the device 200, by including the carrier member 290, provides that many of the parts are universal such that they are compatible with devices of different sizes. For example, the carrier member 290 shown in the FIGURES is designed to be used with fluid displacement chamber 210s which are as large as that shown in the FIGURES or which are smaller than that shown in the FIGURES.

Notwithstanding the fact that using a carrier member 290 provides several advantages, using a carrier member 290 is not imperative to employing the present invention. As discussed above, the present invention can be used in association with, for example, the device disclosed in U.S. Patent No. 5,713,242, which does not include a carriage member.

Benefits derived from the actuating mechanism 246 disclosed herein include elimination of a pair of links, such as disclosed in U.S. Patent Nos. 5,713,242 and 6,796,959, in exchange for a single unitary link 202 that, by virtue of its integrated spring form, is designed to be self-fixturing through its ability to be clipped precisely in position to the mating nut member 204 and thereby retain itself perfectly for receiving insertion of the pivot pin 206 as well as the pin 266 which secures the actuating mechanism 246 to the device 200. With the designs described in U.S. Patent Nos. 5,713,242 and 6,796,959, assembly required alignment and sliding of a first link into its receiving bore followed by up-side-down insertion of a second link onto its receiving bore, then subsequent 180 degree rotation of the second link to align it with the first one. Much more manipulation and thereby assembly time was involved. By comparison, assembly of the actuating mechanism 246 disclosed herein simply involves clipping the unitary link 202 onto the nut member 204, where it retains itself by virtue of its integral leaf spring element 276, and then inserting the pivot pin 206 through both the nut member 204 and the unitary link 202 to hold the assembly together. As discussed above, the pivot pin 206 is preferably made with a reduced diameter middle portion 252 which receives a central rib 254 that is provided in the unitary link 202, which locks the pin 206 in place. This stable, locked together sub-assembly eases subsequent assembly of the inflation device because there are no loose or floppy parts requiring further alignment before the pin 266 is used to mount the actuating mechanism 246 on the device 200. Additionally, the unitary link 202 becomes automatically positioned to receive the pins 206 and 266. In contrast, with the devices disclosed in U.S. Patent Nos. 5,713,242 and 6,796,959, due to the need for free and independent rotation of the pair of links within their respective bores, it was not possible to be certain that these links always stayed in alignment with one another or properly located within the nut member to receive a pin during subsequent assembly of the inflation device. The finished device manufacturer also realizes a distinct advantage through elimination of the detenting mechanism (i.e., the ribs) because obtaining consistent detent resistance is difficult due to detent interference levels being the product of difficult-to-control tolerance variations from three independent parts that become interdependent when assembled.

A significant additional benefit of providing a unitary link 202 in place of a pair of links is the ability of the unitary link 202 to maintain a more precise longitudinal alignment of the nut member 204 to both the pivot pin 206 and the mating threads 279 on the plunger 238. Precise longitudinal alignment was not previously possible with two separate links since each link was free to pivot out of alignment slightly with the other and consequently allow the nut member to slightly twist or deflect out of alignment with the plunger's axis during engagement and disengagement manipulations. Such twist was possible even though plunger and the pin were maintained in strict parallel alignment to one another. This undesirable deflection offers potential for a deleterious effect upon mating tread components resulting in uneven loading and occasional chipping of the most highly-loaded threads during disengagement during maximum pressure use conditions. A unitary link 202 offers far less potential for twisting or deflection compared to two independent links and thereby yields a more robust mechanism.

Another benefit of the actuating mechanism 246 disclosed herein is the design's self-contained nature, its independence from having to react against any additional external structure to obtain thrust necessary to urge the nut member 204 into constant engagement with the plunger 238. Instead, engagement results from thrust of the unitary link's spring element 276 constantly pulling the nut member 204 toward the pin 206 in order to reach a relaxed unstressed condition. This self-contained action simplifies the overall device design and allows the unitary link 202 to co-exist on the assembly line with the earlier, paired link mechanism in order to allow phasing in of the newer design while the older one remains in production. Medical devices are highly regulated and even minor changes must be properly evaluated and validated by each customer before they can be accepted. Due to need for extensive validation work by each and every customer and changes to printed user instructions, wholesale changeover to a new mechanism is not possible and there will be a period of time where both constructions must be manufactured at the same time as customers switch over, one by one. Not having to change the larger device structure that houses this mechanism reduces overall tooling expense, eliminates processing changes and makes process and component revalidations unnecessary for the device housing 208 structure thereby simplifying and speeding market introduction of the new design.

As discussed above, Figures 4-6 illustrate a fluid displacement device 200 which is in accordance with a specific example (not claimed), and that device 200 has been described in detail hereinabove. Figures 19-20 illustrate a fluid displacement device 200a which is in accordance with an embodiment of the present invention as claimed. This embodiment has many of the same parts. As such, identical references numbers are used, and a detailed discussion of those parts is going to be omitted when discussing the embodiment shown in Figures 19-20.

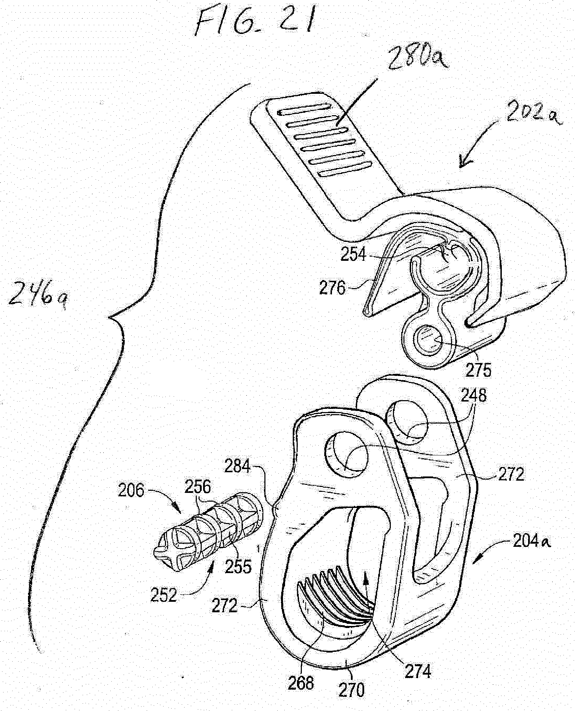

The only difference between the two devices (i.e., fluid displacement device 200 and fluid displacement device 200a) is that instead of providing a control surface in the form of a lever 280 on a nut member 204, the control surface of the fluid displacement device 200a is provided in the form of a lever 280a on a unitary link 202a. As such, the only difference between the fluid displacement device 200 shown in Figure 4-6 and the fluid displacement device 200a shown in Figures 19-20 is that the unitary link 202a and the nut member 204a of the fluid displacement device 200a are shaped differently than the unitary link 202 and the nut member 204 of the fluid displacement device 200. The preferred shape and configuration of the unitary link 202a and the nut member 204a are shown in Figures 19-20, and Figure 21 illustrates the actuating mechanism 246a that is provided by the unitary link 202a, the nut member 204a, and the pivot pin 206.

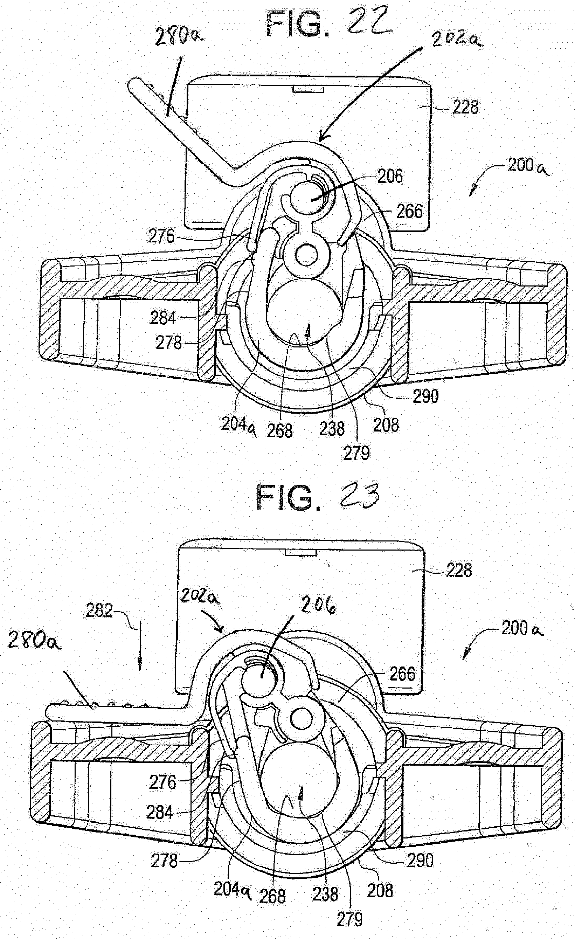

In operation, the fluid displacement device 200a shown in Figures 19-20 works effectively the same as the fluid displacement device 200 shown in Figures 4-6 and previously described, except that instead of pressing on a control surface in the form of a lever 280 on a nut member 204 to toggle the device (as is performed in associate with fluid displacement device 200), fluid displacement device 200a provides that one presses on a control surface in the form of a lever 280a on the unitary link 202a in order to toggle the device. While Figure 22 shows the nut member 204a biased into engagement with the plunger 238, Figure 23 shows the nut member 204a moved out of engagement with the plunger 238.

It is more preferable to provide the control surface on the unitary link (as is provided in association with the fluid displacement device 200a) than to provide the control surface on the nut member (as is provided in association with the fluid displacement device 200) because it simplifies the design and therefore the tooling of the nut member for easier manufacture of the nut member while having minimal impact upon the design and ease of manufacture of the unitary link.

Figure 24 illustrates an alternative actuating mechanism 246b that can be employed instead of either one of the actuating mechanisms 246, 246a previously described, preferably in association with a fluid displacement device having all the same parts as fluid displacement device 200 or 200a. Like the actuating mechanisms 246, 246a previously described, the actuating mechanism 246b shown in Figure 24 includes a nut member 204b and a pivot pin 206. While the nut member 204b is exactly the same as nut member 204a, the unitary link 204b is different from unitary link 204a. Specifically, instead of providing a control surface in the form of a lever 280a as is provided on unitary link 202a, the unitary link 202b shown in Figure 24 provides a control surface in the form of a surface of the unitary link 202b.

In operation, a fluid displacement device which employs the actuating mechanism 246b shown in Figure 24 works much the same way as the fluid displacement devices 200, 200a previously described, except that instead of pressing on a control surface in the form of a lever 280 on a nut member 204 or a lever 280a on a unitary link 202a, the control surface 280b is pressed to actuate the device.

While specific embodiments of the present invention are shown and described, it is envisioned that those skilled in the art may devise various modifications of the present invention without departing from the scope of the foregoing disclosure.

## Claims

1. A toggle locking mechanism (246) for a fluid displacement device which has a housing (208) and a plunger (238) which extends into the housing (208), said toggle locking mechanism (246) having a nut member (204b) and a unitary link (202b), **characterized in that** the unitary link (202b) has a control surface (280b) which is pressable to cause the nut member (204b) to disengage the plunger (238).

2. A toggle locking mechanism (246) of claim 1, wherein the nut member (204b) has threads (268) which are biased into engagement with threads on the plunger, but which is selectively disenangageable with the threads (279) on the plunger (238).

3. A toggle locking mechanism (246) of claims 1-2, wherein a pivot pin (206) secures the unitary link (202b) to the nut member (204b).

4. A toggle locking mechanism (246) of claims 1-3, wherein a rib (254) on the unitary link (202b) engages and secures the pivot pin (206) relative to the unitary link (202b).

5. A toggle locking mechanism (246) of claims 1-4, wherein the unitary link (202b) has a spring finger (276) which contacts the external surface (278) of the nut member (204b).

6. A toggle mechanism (246) of claim 5, wherein the spring finger (276) is configured to provide that the nut member (204b) is biased into engagement with the plunger (238).

7. A toggle locking mechanism (246) as recited in claim 2, **characterized in that** the link member has a spring finger (276) which contacts an external surface (278) of the nut member (204b).

8. A toggle locking mechanism (246) as recited in claim 7, **characterized in that** the spring finger (276) is configured to provide that the nut member (204b) is biased into engagement with the plunger (238).

9. A toggle locking mechanism (246) as recited in claim 8, **characterized in that** the spring finger (276) contacts a cam surface (284) on the nut member (204b) and provides at least one of auditory and tactile feedback regarding whether the nut member (204b) is engaged with the plunger (238).

10. A fluid displacement device comprising a housing (208), a plunger (238) which extends into the housing (208), and a toggle locking mechanism (246) as claimed in any one of claims 1 to 9.

11. A method of assembling a fluid displacement device which has a plunger (238), a unitary link member, and a nut member (204b), said method comprising assembling an actuating mechanism (246) by securing the unitary link member to the nut member (204b), and securing the actuating mechanism (246) to the fluid displacement device, said method **characterized by** actuating the actuating mechanism (246) by pressing a control surface (280b) on the unitary link member, sticking the plunger (238) through the actuating mechanism (246); and de-actuating the actuating mechanism (246) by releasing the control surface (280b) on the unitary link member wherein the nut member (204b) engages the plunger (238).

12. A method as recited in claim 11, **characterized in that** the step of assembling the actuating mechanism (246) comprises engaging the unitary link member with the nut member (204b), wherein the unitary link member tends to stay engaged with the nut member (204b) due to a spring finger portion (276) of the unitary link member contacting an external surface (278) of the nut member (204b), and using a pivot pin (206) to secure the unitary link member to the nut member (204b).

13. A method as recited in claim 12, **characterized in that** the step of using the pivot pin (206) to secure the unitary link member to the nut member (204b) comprises having a rib (254) on the unitary link member engage and secure the pivot pin (206) relative to the link member.

## Patentansprüche

1. Kipphebelverriegelungsmechanismus (246) für eine Flüssigkeitsverdrängungsvorrichtung, der ein Gehäuse (208) und einen Stößel (238) aufweist, der sich in das Gehäuse (208) erstreckt, wobei der Kipphebelverriegelungsmechanismus (246) ein Mutterelement (204b) und ein einstückiges Verbindungsglied (202b) aufweist, **dadurch gekennzeichnet, dass** das einstückige Verbindungsglied (202b) eine Steuerfläche (280b) aufweist, die gedrückt werden kann, um zu bewirken, dass das Mutterelement (204b) den Stößel (238) außer Eingriff bringt.

2. Kipphebelverriegelungsmechanismus (246) nach Anspruch 1, wobei das Mutterelement (204b) Gewinde (268) aufweist, die in einen Eingriff mit den Gewinden auf dem Stößel vorgespannt sind, aber gezielt außer Eingriff mit den Gewinden (279) auf dem Stößel (238) gebracht werden können.

3. Kipphebelverriegelungsmechanismus (246) nach Anspruch 1 bis 2, wobei ein Gelenkstift (206) das einstückige Verbindungsglied (202b) an dem Mutterelement (204b) sichert.

4. Kipphebelverriegelungsmechanismus (246) nach Anspruch 1 bis 3, wobei eine Rippe (254) an dem einstückigen Verbindungsglied (202b) in Eingriff mit dem Gelenkstift (206) gelangt und diesen relativ zu dem einstückigen Verbindungsglied (202b) sichert.

5. Kipphebelverriegelungsmechanismus (246) nach Anspruch 1 bis 4, wobei das einstückige Verbindungsglied (202b) einen Federfinger (276) aufweist, der die Außenfläche (278) des Mutterelements (204b) berührt.

6. Kipphebelverriegelungsmechanismus (246) nach Anspruch 5, wobei der Federfinger (276) dazu ausgelegt ist, vorzusehen, dass das Mutterelement (204b) in einen Eingriff mit dem Stößel (238) vorgespannt ist.

7. Kipphebelverriegelungsmechanismus (246) nach Anspruch 2, **dadurch gekennzeichnet, dass** das Verbindungsgliedelement einen Federfinger (276) aufweist, der eine Außenfläche (278) des Mutterelements (204b) berührt.

8. Kipphebelverriegelungsmechanismus (246) nach Anspruch 7, **dadurch gekennzeichnet, dass** der Federfinger (276) dazu ausgelegt ist, vorzusehen, dass das Mutterelement (204b) in einen Eingriff mit dem Stößel (238) vorgespannt ist.

9. Kipphebelverriegelungsmechanismus (246) nach Anspruch 8, **dadurch gekennzeichnet, dass** der Federfinger (276) eine Nockenfläche (284) an dem Mutterelement (204b) berührt und entweder eine akustische oder eine fühlbare Rückmeldung darüber bereitstellt, ob sich das Mutterelement (204b) in Eingriff mit dem Stößel (238) befindet.

10. Flüssigkeitsverdrängungsvorrichtung, die ein Gehäuse (208), einen sich in das Gehäuse (208) erstreckenden Stößel (238) und einen Kipphebelverriegelungsmechanismus (246) nach einem der Ansprüche 1 bis 9 umfasst.

11. Verfahren zum Zusammenbauen einer Flüssigkeitsverdrängungsvorrichtung, die einen Stößel (238), ein einstückiges Verbindungsgliedelement und ein Mutterelement (204b) aufweist, wobei das Verfahren das Zusammenbauen eines Betätigungsmechanismus (246) durch Sichern des einstückigen Verbindungsgliedelements an dem Mutterelement (204b) und das Sichern des Betätigungsmechanismus (246) an der Flüssigkeitsverdrängungsvorrichtung umfasst, wobei das Verfahren **gekennzeichnet ist durch** das Betätigen des Betätigungsmechanismus (246) durch Drücken einer Steuerfläche (280b) an dem einstückigen Verbindungsgliedelement, das Stecken des Stößels (238) durch den Betätigungsmechanismus (246) und das Deaktivieren des Betätigungsmechanismus (246) durch Loslassen der Steuerfläche (280b) an dem einstückigen Verbindungsgliedelement, wobei das Mutterelement (204b) in Eingriff mit dem Stößel (238) gelangt.

12. Verfahren nach Anspruch 11, **dadurch gekennzeichnet, dass** der Schritt des Zusammenbauens des Betätigungsmechanismus (246) das Ineingriffbringen des einstückigen Verbindungsgliedelements mit dem Mutterelement (204b), wobei das einstückige Verbindunsgliedelement dazu neigt, in Eingriff mit dem Mutterelement (204b) zu verbleiben, da ein Federfingerabschnitt (276) des einstückigen Verbindungsgliedelements eine Außenfläche (278) des Mutterelements (204b) berührt, und das Verwenden eines Gelenkstifts (206) umfasst, um das einstückige Verbindungsgliedelement an dem Mutterelement (204b) zu sichern.

13. Verfahren nach Anspruch 12, **dadurch gekennzeichnet, dass** der Schritt des Verwendens des Gelenkstifts (206), um das einstückige Verbindungsgliedelement an dem Mutterelement (204b) zu sichern, das Bewirken umfasst, dass eine Rippe (254) an dem einstückigen Verbindungsgliedelement in Eingriff mit dem Gelenkstift (206) gelangt und diesen relativ zu dem Verbindungsgliedelement sichert.

## Revendications

1. Mécanisme de verrouillage à genouillère (246) pour un dispositif de déplacement de fluide qui comprend un boîtier (208) et un plongeur (238) qui s'étend dans le boîtier (208), ledit mécanisme de verrouillage à genouillère (246) comprenant un élément d'écrou (204b) et une biellette unitaire (202b), **caractérisé en ce que** la biellette unitaire (202b) présente une surface de commande (280b) qui peut être pressée pour amener l'élément d'écrou (204b) à désengager le plongeur (238) .

2. Mécanisme de verrouillage à genouillère (246) selon la revendication 1, dans lequel l'élément d'écrou (204b) comporte des filets (268) qui sont poussés en engagement avec des filets sur le plongeur, mais qui peuvent être désengagés de façon sélective des filets (279) sur le plongeur (238).

3. Mécanisme de verrouillage à genouillère (246) selon les revendications 1 et 2, dans lequel un axe d'articulation (206) fixe la biellette unitaire (202b) à l'élément d'écrou (204b).

4. Mécanisme de verrouillage à genouillère (246) selon les revendications 1 à 3, dans lequel une nervure (254) sur la biellette unitaire (202b) engage et fixe l'axe d'articulation (206) par rapport à la biellette unitaire (202b).

5. Mécanisme de verrouillage à genouillère (246) selon les revendications 1 à 4, dans lequel la biellette unitaire (202b) comprend un doigt à ressort (276) qui est en contact avec la surface externe (278) de l'élément d'écrou (204b).

6. Mécanisme de verrouillage à genouillère (246) selon la revendication 5, dans lequel le doigt à ressort (276) est configuré de manière à assurer que l'élément d'écrou (204b) soit poussé en engagement avec le plongeur (238).

7. Mécanisme de verrouillage à genouillère (246) selon la revendication 2, **caractérisé en ce que** l'élément de biellette comprend un doigt à ressort (276) qui est en contact avec une surface externe (278) de l'élément d'écrou (204b).

8. Mécanisme de verrouillage à genouillère (246) selon la revendication 7, **caractérisé en ce que** le doigt à ressort (276) est configuré de manière à assurer que l'élément d'écrou (204b) soit poussé en engagement avec le plongeur (238).

9. Mécanisme de verrouillage à genouillère (246) selon la revendication 8, **caractérisé en ce que** le doigt à ressort (276) est en contact avec une surface de came (284) sur l'élément d'écrou (204b) et génère au moins un retour sonore ou tactile pour indiquer si l'élément d'écrou (204b) est engagé avec le plongeur (238).

10. Dispositif de déplacement de fluide comprenant un boîtier (208), un plongeur (238) qui s'étend dans le boîtier (208), et un mécanisme de verrouillage à genouillère (246) selon l'une quelconque des revendications 1 à 9.

11. Procédé d'assemblage d'un dispositif de déplacement de fluide comprenant un plongeur (238), un élément de biellette unitaire et un élément d'écrou (204b), ledit procédé comprenant l'assemblage d'un mécanisme d'actionnement (246) en fixant l'élément de biellette unitaire à l'élément d'écrou (204b), et la fixation du mécanisme d'actionnement (246) au dispositif de déplacement de fluide, ledit procédé étant **caractérisé par** l'actionnement du mécanisme d'actionnement (246) en pressant une surface de commande (280b) sur l'élément de biellette unitaire, en enfonçant le plongeur (238) à travers le mécanisme d'actionnement (246); et en désactivant le mécanisme d'actionnement (246) en relâchant la surface de commande (280b) sur l'élément de biellette unitaire, dans lequel l'élément d'écrou (204b) engage le plongeur (238) .

12. Procédé selon la revendication 11, **caractérisé en ce que** l'étape d'assemblage du mécanisme d'actionnement (246) comprend l'engagement de l'élément de biellette unitaire avec l'élément d'écrou (204b), dans lequel l'élément de biellette unitaire a tendance à rester engagé avec l'élément d'écrou (204b) en raison d'une partie de doigt à ressort (276) de l'élément de biellette unitaire qui est en contact avec une surface externe (278) de l'élément d'écrou (204b), et l'utilisation d'un axe d'articulation (206) pour fixer l'élément de biellette unitaire à l'élément d'écrou (204b) .

13. Procédé selon la revendication 12, **caractérisé en ce que** l'étape d'utilisation de l'axe d'articulation (206) pour fixer l'élément de biellette unitaire à l'élément d'écrou (204b) inclut qu'une nervure (254) sur l'élément de biellette unitaire engage et fixe l'axe d'articulation (206) par rapport à l'élément de biellette.
